Europäisches Patentamt

**European Patent Office**

**Office européen des brevets**

(19)

(11) Publication number: **0 211 676 B1**

## EUROPEAN PATENT SPECIFICATION

(12)

(45) Date of publication of patent specification: **11.12.91**

(51) Int. Cl.⁵: **A61F 2/30, A61C 8/00, A61L 27/00**

(21) Application number: **86306174.3**

(22) Date of filing: **08.08.86**

(54) Method for producing endosseous implants.

(30) Priority: **08.08.85 JP 175569/85**

(43) Date of publication of application:
**25.02.87 Bulletin 87/09**

(45) Publication of the grant of the patent:
**11.12.91 Bulletin 91/50**

(84) Designated Contracting States:
**CH DE FR GB IT LI SE**

(56) References cited:
**DE-A- 2 810 129**
**DE-A- 2 818 630**
**FR-A- 2 318 617**
**FR-A- 2 336 913**
**FR-A- 2 374 019**

(73) Proprietor: **SUMITOMO CHEMICAL COMPANY, LIMITED**
**Kitahama 4-chome 5-33**
**Chuo-ku Osaka 541(JP)**

(72) Inventor: **Suzuki, Kazuo**
**2135-5, Hirooka Takade**
**Shiojiri-shi Nagano-ken(JP)**
Inventor: **Ito, Mitsuo**
**1563, Hirooka Takade**
**Shiojiri-shi Nagano-ken(JP)**

(74) Representative: **Bizley, Richard Edward et al**
**BOULT, WADE & TENNANT 27 Furnival Street**
**London EC4A 1PQ(GB)**

Rank Xerox (UK) Business Services

**Description**

This invention relates to endosseous implants and to a method for the production thereof.

Implantation technology, which comprises the insertion of artificial materials such as artificial organs, artificial blood vessels, artificial joints, artificial bones and artificial tooth roots into the body, has attracted much attention in recent years. It is said that trials of implantation go back to ancient times. Particularly in the last ten years a huge number of treatments by implantation have been performed on bones and tooth roots to afford good results in the remedy of the defects or the recovery of functions thereof. However, there has not yet been obtained an artificial bone or tooth root which satisfies the necessary requirements as a material for living bodies, i.e. affinity to living tissue, safety and excellent durability.

As metallic materials which have mainly been used for preparation of artificial bones or tooth roots, cobalt-chromium alloys, stainless steel, titanium and tantalum are exemplified. On the other hand, as ceramic materials, alumina or materials comprising predominantly carbon have been recently taken note of.

Although metallic materials are excellent in mechanical strength, particularly in impact strength, they lack affinity for living tissue. For example, when a metallic implant is used, metal ions are dissolved out therefrom and are toxic to bone cells around the implant. Furthermore, bone-formation is obstructed, probably because of too large a thermal conductivity of the metallic implant. Among the metallic materials, titanium and tantalum are particularly superior in a corrosion-resistance and hence have been employed as fixing plates for skulls or fractured parts of bones and implants for jawbones since about 1940, but these are not necessarily satisfactory.

On the other hand, ceramic materials generally show a good affinity to bones, and tissues penetrate into fine pores of ceramic materials to afford a strong fixation, without adverse reaction between the ceramic material and the tissue. Besides, they are also excellent in durability, that is, they are resistant to corrosion or decomposition. On the other hand, they possess a poor impact strength.

There has been proposed an implant having the characteristics of both metallic materials and ceramic materials, i.e. an implant prepared by thermally spraying a ceramic material onto the surface of a metallic core material (cf. Japanese Patent First Publication Nos 14095/1977, 82893/1977, 28997/1978 and 75209/1978). In these methods, however, a self-bonding type bonding agent is used in order to improve the adhesion of the ceramic coating layer. The bonding agent has the problem that it contains nickel, chromium, etc. which dissolve out in living tissue and have toxic effects on the body.

FR-A-2 336 913 also describes a method of thermally spraying ceramic material onto the surface of a metallic core material but in this method also a bonding agent is firstly applied.

According to the present invention there is provided a method for producing endosseous implants comprising thermally spraying a ceramic material onto the surface of a metallic titanium core material, wherein the improvement comprises subjecting the metallic titanium core material (6) to a surface oxidation treatment by heating the core material at a temperature of 400° to 800°C in air then thermally spraying the ceramic material (7,8) onto the surface of the metallic titanium core material, wherein the ceramic material is aluminum oxide, hydroxyapatite or a mixture thereof.

The present invention is further described by way of example only with reference to the accompanying drawings, in which:

Figure 1 is a schematic view of an embodiment of an endosseous implant for the lower jawbone of a dog, wherein 1 represents the lower jawbone, 2 and 3 are natural teeth, 4 is an artificial tooth root and 5 is an artificial tooth crown attached on the artificial tooth root 4.

Figure 2 is a schematic view of an embodiment of a blade type endosseous implant for jawbone and (A) is a front view thereof and (B) is a side view thereof, wherein 6 represents a surface-oxidized metallic titanium core material and 7 and 8 are each a thermally plasma sprayed layer comprising predominantly ceramics.

Figure 3 is a graph showing the correlation between the heating temperature of titanium material and the adhesion strength of the coating layer formed by the thermal plasma spray.

Figure 4 is a graph showing the correlation between the heating temperature of titanium material and the change of elastic modulus of the titanium material.

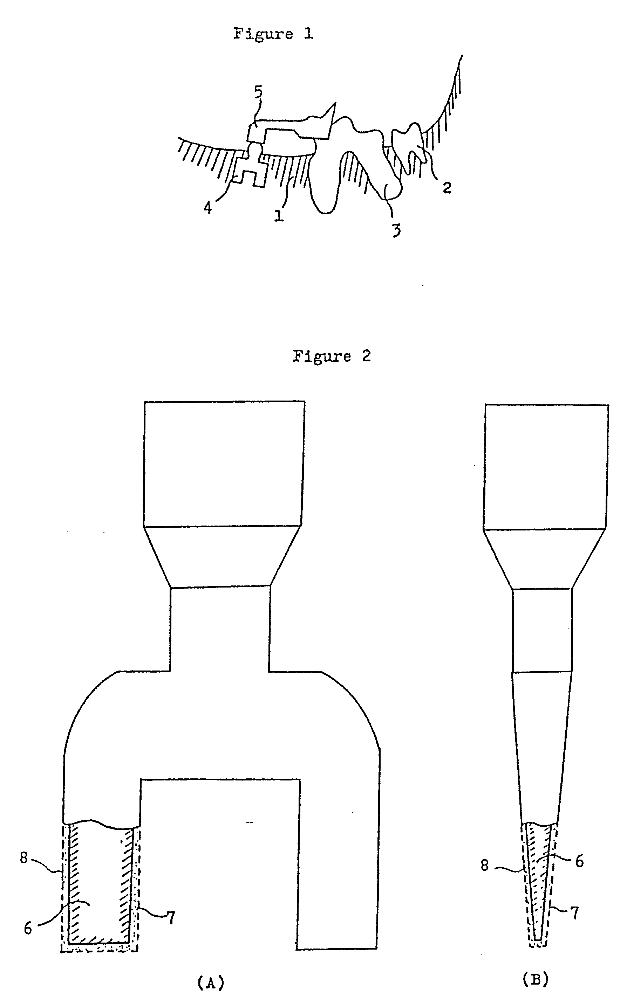

According to the present invention, as is shown in Figure 2, a ceramic coating is applied to the surface of a metallic implant core material so as to obtain an implant with good impact strength and interacting with the surrounding bone tissues in a similar manner to ceramic materials.

The metallic core materials used in this invention may be any suitable titanium materials which have appropriate mechanical strength and are not harmful to the body, including those titanium materials which have usually been used as artificial materials for bones, joints and tooth roots which do not exhibit harmful effects on living bodies and possess an appropriate mechanical strength, for example, titanium and titanium alloys (e.g. 6 % Al-4 % V-Ti, etc.).

The ceramic materials used in this invention may be, for example, hydroxyapatite, calcium phosphate, aluminum oxide, zirconium oxide or titanium oxide, which may be used alone or in combination of two or more thereof. In order to control the pores in the ceramic layer, a porcelain may be applied by thermally spraying it together with the ceramic material or by baking on the ceramic coating layer. For such a purpose, there can be used porcelains such as Dentin and Enamel (Trade Marks), for example. Among the ceramic materials, preferred are hydroxyapatite and aluminum oxide in view of their excellent affinity with living tissue. A combination of hydroxyapatite and aluminum oxide is particularly suitable because it interacts most intimately with living tissue.

The endosseous implants of this invention can be produced in the following manner.

The metallic material is formed into the desired shape, for example by conventional methods, such as cutting, casting, forging, punching, electro arc machining, laser-processing, or powdered metal technique. The surface of the metallic titanium core material thus formed may be roughened, for example, by mechanical methods such as grinding, sandblasting, grit blasting, etc.; chemical etching such as treatment with an acid or alkali; electrolytic etching; and the like, prior to being subjected to the surface oxidation treatment.

The surface oxidation treatment of the metallic titanium core material can be carried out by various methods, for example, by heat-treatment in air or an anodizing process, but preferably by heat-treatment in air. The heat-treatment is preferably done at a temperature of 400 to 800°C. When the temperature is lower than 400°C, the ceramic coating layer formed by the thermal spray tends to show inferior adhesion. On the other hand, when the temperature is higher than 800°C, the strength of the materials tends to deteriorate and further the surface oxide layer tends to become too thick which causes lowering of adhesion of the coating layer. A most preferred heating temperature is in the range of 450 to 550°C. in view of the excellent adhesion of the coating layer and the strength of the materials which can be obtained.

The heat-treating period of time is not critical, but is preferably in the range of 1 to 100 minutes for practical reasons. The heat-treatment of the metallic titanium core material is usually carried out in a conventional electric furnace or gas furnace.

In the thermal spraying of ceramic materials. any portion which is not coated with the ceramic material is previously masked by an appropriate means, for instance, application of a marking ink or an aluminum adhesive tape prior to the treatment for roughening the surface. The thermal spraying of the ceramic material is preferably carried out by a thermal plasma spraying apparatus. Some portions of the endosseous implants, for instance, the ceramic coating layer in artificial joints, are required to be highly smooth. In such a case, a porcelain is coated onto the surface and the coated product is repeatedly calcined in a vacuum furnace.

In the endosseous implants of this invention, the thickness of the ceramic coating layer which optionally contains porcelain is not particularly limited, but is preferably in the range of 10 to 200 μm.

This invention is illustrated by the following Examples but should not be construed to be limited thereto.

Example

A core material for an endosseous implant is prepared from a titanium material (JIS, second class of material) by cutting and grinding the titanium material by electro arc machining.

The metallic core material for the implant is grit-blasted with a blast apparatus [a mammoth type ventiblast apparatus, manufactured by Metco Inc., England: blasting agent: Metcolite VF, manufactured by Metco Inc.; pressure: 30 psi (205 kPa)].

The blasted core material is heat-treated at 500°C for 10 minutes. Thereafter, under an argon-hydrogen-plasma jet flame (ARC electric current 500 Amp) generated by a plasma spray apparatus (6MM-630 type, manufactured by Metco Inc., equipped with an electric power supplier), a ground mixture of hydroxyapatite (particle size: 10 - 100 μm, 80 % by weight) and aluminum oxide (WA #120, manufactured by Nippon Kenmazai K.K., 20 % by weight) is thermally sprayed to form a coating layer having a thickness of about 150 μm on average. The thermally sprayed coating layer has excellent adhesion, and even when the product is subjected to bending processing at an angle of 160°, the coating layer is not peeled off.

The product obtained above was tested as follows:

The implant was embedded into the lower jawbone of a dog. After 3 months, it was observed by X-ray fluoroscopy. As a result, formation of dense bone around the implant was confirmed.

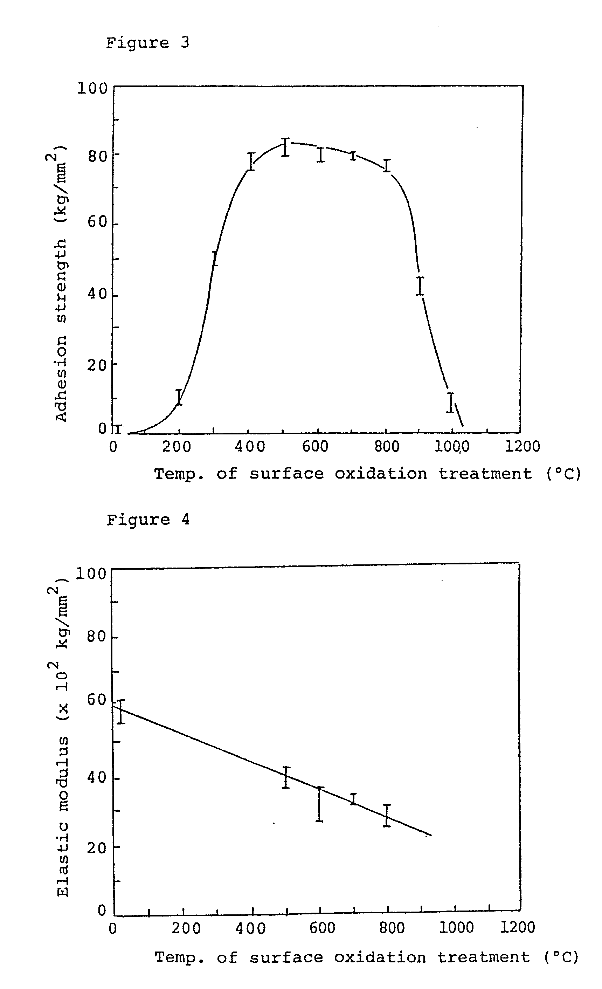

The correlation of the temperature in the heat-treatment with the adhesion of the coating layer and also with the elastic modulus of the core material are shown in the accompanying Fig. 3 and Fig. 4, respectively, wherein the data in the following Reference Example are also shown. The sample (width 5 mm x thickness 1 mm x length 50 mm) used in the test was prepared from the same material as used in the Example in the same manner. The adhesion of the coating layer and the elastic modulus of the core material were measured by a three-point bending test where the sample was kept at a span distance of 30 mm. As is clear from Fig. 3 and Fig. 4, the temperature for the heat-treatment is preferably in the range of 400 to 800°C.

Reference Example

A core material for an endosseous implant is prepared by treating the same titanium material in the same manner as described in the Example. The core material is likewise subjected to grit blasting, but is not subjected to heat-treatment.

The blasted core material is thermally sprayed with a powdery mixture of titanium oxide and aluminum oxide in a layer having a thickness of about 50 μm on average as the first coating layer, and then further thermally sprayed thereon with a mixture of hydroxyapatite and aluminum oxide in a layer having a thickness of about 150 μm on average as the second coating layer. The resulting product has significantly inferior adhesion of the coating layer and the coating layer is easily peeled off even by a light impact. This product cannot be used as an endosseous implant.

It will be appreciated from the foregoing that in accordance with the invention there may be provided endosseous implants having a core of metallic titanium material with an oxidised surface and a ceramic material directly bonded to the core without a bonding agent.

Thus, according to the present invention, by thermally spraying a ceramic material on the surface of a metallic titanium core material which is surface-oxidized, there can be produced excellent endosseous implants which improve the defect of ceramic implants of being easily breakable whilst keeping the excellent characteristics of the ceramic material and have the excellent mechanical strength of the metallic material and further can interact with the surrounding bone tissues in a similar manner to ceramic material.

**Claims**

1. A method for producing endosseous implants comprising thermally spraying a ceramic material onto the surface of a metallic titanium core material, wherein the improvement comprises subjecting the metallic titanium core material (6) to a surface oxidation treatment by heating the core material at a temperature of 400° to 800°C in air then thermally spraying the ceramic material (7,8) onto the surface of the metallic titanium core material, wherein the ceramic material is aluminum oxide, hydroxyapatite or a mixture thereof.

2. A method as claimed in claim 1, wherein the temperature for the surface oxidation treatment is in the range of 450 to 550°C.

3. A method as claimed in claim 1 or claim 2, wherein the thermal spraying is carried out by thermal plasma spraying.

4. A method as claimed in any one of the preceding claims, wherein the surface oxidation treatment is conducted for 1 to 100 minutes.

5. A method as claimed in any one of the preceding claims, wherein the thickness of the ceramic coating layer (7,8) is from 10 to 200 μm.

6. A method as claimed in any one of the preceding claims, wherein a ground mixture of hydroxyapatite and aluminum oxide is thermally sprayed to form a coating layer (7,8) having an average thickness of about 150 μm.

**Revendications**

1. Procédé de production d'implants intra-osseux, comprenant la pulvérisation à chaud d'un matériau céramique sur la surface d'un matériau de noyau métallique en titane, dans lequel l'amélioration comprend la soumission du matériau du noyau métallique en titane (6) à un traitement oxydant superficiel par chauffage du matériau du noyau à une température comprise entre 400 et 800°C à l'air, puis la pulvérisation à chaud du matériau céramique (7,8) sur la surface du matériau du noyau métallique en titane, le matériau céramique étant de l'oxyde de l'aluminium, de l'hydroxyapatite ou un mélange de ces substances.

2. Procédé selon la revendication 1, dans lequel la température du traitement superficiel oxydant est comprise dans la plage de 450 à 550°C.

3. Procédé selon la revendication 1 ou la revendication 2, dans lequel la pulvérisation à chaud est effectuée par pulvérisation à chaud de plasma.

4. Procédé selon l'une quelconque des revendications précédentes, dans lequel le traitement superficiel oxydant est mené pendant 1 à 100 min.

5. Procédé selon l'une quelconque des revendications précédentes, dans lequel l'épaisseur de la couche de revêtement céramique (7,8) est comprise entre 10 et 200 μm.

6. Procédé selon l'une quelconque des revendications précédentes, dans lequel un mélange broyé d'hydroxyapatite et d'oxyde d'aluminium est pulvérisé à chaud pour former une couche de revêtement (7,8) ayant une épaisseur moyenne d'environ 150 µm.

## Patentansprüche

1. Verfahren zur Herstellung endossaler Implantate mittels thermischen Aufsprühens eines keramischen Materials auf die Oberfläche eines metallischen Titankernmaterials, wobei die Verbesserung eine Oberflächenoxidationsbehandlung des metallischen Titankernmaterials (6) durch Erwärmen des Kernmaterials auf eine Temperatur von 400° bis 800°C in Luft und anschließend thermisches Aufsprühen des keramischen Materials (7, 8) auf die Oberfläche des metallischen Titankernmaterials aufweist, wobei das Keramikmaterial Aluminiumoxid, Hydroxylapatit oder eine Mischung aus diesen ist.

2. Verfahren nach Anspruch 1, wobei die Temperatur für die Oberflächenoxidationsbehandlung im Bereich von 450 bis 550°C liegt.

3. Verfahren nach Anspruch 1 oder Anspruch 2, wobei das thermische Aufsprühen durch thermisches Plasmasprühen durchgeführt wird.

4. Verfahren nach einem der vorstehenden Ansprüche, wobei die Oberflächenoxidationsbehandlung 1 bis 100 Minuten lang durchgeführt wird.

5. Verfahren nach einem der vorstehenden Ansprüche, wobei die Dicke der keramischen Beschichtungslage (7, 8) 10 bis 200 µm beträgt.

6. Verfahren nach einem der vorstehenden Ansprüche, wobei eine Grundmischung aus Hydroxylapatit und Aluminiumoxid thermisch aufgesprüht wird, so daß eine Beschichtungslage (7, 8) mit einer mittleren Dicke von etwa 150 µm gebildet wird.

Figure 1

Figure 2

(A)

(B)

Figure 3

Figure 4